(19) **Europäisches Patentamt**
**European Patent Office**
**Office européen des brevets**

(11) **EP 1 402 044 B1**

(12) **EUROPÄISCHE PATENTSCHRIFT**

(45) Veröffentlichungstag und Bekanntmachung des Hinweises auf die Patenterteilung:
**12.08.2009  Patentblatt 2009/33**

(21) Anmeldenummer: **02740678.4**

(22) Anmeldetag: **04.06.2002**

(51) Int Cl.:
*C12P 7/20* (2006.01)     *C12P 7/64* (2006.01)
*C12P 7/62* (2006.01)     *C12M 1/00* (2006.01)
*C12M 1/33* (2006.01)     *C11C 1/04* (2006.01)
*C11C 3/00* (2006.01)     *C11C 3/10* (2006.01)

(86) Internationale Anmeldenummer:
**PCT/EP2002/006077**

(87) Internationale Veröffentlichungsnummer:
**WO 2003/010323 (06.02.2003 Gazette 2003/06)**

(54) **VERFAHREN UND VORRICHTUNG ZUR GEWINNUNG VON FETTSÄUREESTERN AUS NATIVEN ÖLEN UND FETTEN DURCH DEREN ENZYMATISCHE SPALTUNG**

METHOD AND DEVICE FOR OBTAINING FATTY ACID ESTERS FROM NATIVE OILS AND FATS BY MEANS OF ENZYMATIC CLEAVAGE THEREOF

PROCEDE ET DISPOSITIF POUR OBTENIR DES ESTERS D'ACIDES GRAS A PARTIR D'HUILES ET DES GRAISSES NATURELLES PAR LEUR DIGESTION ENZYMATIQUE

(84) Benannte Vertragsstaaten:
**AT BE CH CY DE DK ES FI FR GB GR IE IT LI LU MC NL PT SE TR**
Benannte Erstreckungsstaaten:
**RO SI**

(30) Priorität: **21.06.2001  EP 01115081**
**20.11.2001  DE 10156584**

(43) Veröffentlichungstag der Anmeldung:
**31.03.2004  Patentblatt 2004/14**

(73) Patentinhaber: **T + T Oleochemie GmbH**
**63755 Alzenau (DE)**

(72) Erfinder:
• **BRUNNER, Karlheinz**
**63538 Grosskrotzenburg (DE)**
• **FRISCHE, Rainer**
**60529 Frankfurt (DE)**
• **RICKER, Rainer**
**63128 Dietzenbach (DE)**
• **KASKE, Corinna**
**63736 Aschaffenburg (DE)**
• **KILIAN, Dirk**
**63477 Maintal (DE)**

(74) Vertreter: **Englaender, Klaus et al**
**HML**
**Patentanwälte**
**Schraudolphstrasse 3**
**80799 München (DE)**

(56) Entgegenhaltungen:
**EP-A- 0 195 311          DE-A- 19 922 882**

• **BUEHLER M ET AL: "Continuous use of lipases in fat hydrolysis" FETT - LIPID, WILEY-VCH VERLAG,WEINHEIM, DE, Bd. 89, Nr. 14, 1987, Seiten 598-605, XP002188305 ISSN: 0931-5985 in der Anmeldung erwähnt**
• **DATABASE BIOSIS [Online] BIOSCIENCES INFORMATION SERVICE, PHILADELPHIA, PA, US; 1993 TANIGAKI MASANOBU ET AL: "Hydrolysis of soybean oil by lipase with a bioreactor having two different membranes." Database accession no. PREV199395084843 XP002182853 & JOURNAL OF FERMENTATION AND BIOENGINEERING, Bd. 75, Nr. 1, 1993, Seiten 53-57, ISSN: 0922-338X**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- GAN Q ET AL: "SIMULTANEOUS REACTION AND SEPARATION IN ENZYMATIC HYDROLYSIS OF HIGH OLEATE SUNFLOWER OIL - EVALUATION OF ULTRAFILTRATION PERFORMANCE AND PROCESS SYNERGY" CHEMICAL ENGINEERING JOURNAL, ELSEVIER SEQUOIA, LAUSANNE, CH, Bd. 71, Nr. 2, 21. Dezember 1998 (1998-12-21), Seiten 87-96, XP001022886 ISSN: 1385-8947

- PATENT ABSTRACTS OF JAPAN vol. 013, no. 111 (C-577), 16. März 1989 (1989-03-16) & JP 63 287492 A (KAO CORP), 24. November 1988 (1988-11-24)

**Beschreibung**

Die Erfindung betrifft ein Verfahren und eine Vorrichtung zur Gewinnung von Fettsäuren oder Fettsäureestern aus nativen Ölen und Fetten durch deren enzymatische Spaltung und wahlweise gleichzeitig stattfindende enzymatische Esterbildung unter Einsatz von Alkoholen, insbesondere n- und Iso-Alkoholen.

Die enzymatische Spaltung von Ölen und Fetten ist zwar seit langem bekannt, hat sich jedoch wegen des hohen Enzymverbrauchs bisher gegenüber der Druckspaltung nicht durchgesetzt.

Die Fettsäureester aus nativen, in Ölen und Fetten enthaltenen Fettsäuren und mittelkettigen (ab einer Kettenlänge C6) bis langkettigen (im allgemeinen bis zu einer Kettenlänge C-24 ) n- und Iso-Alkoholen haben für zahlreiche Anwendungen speziell auf dem Schmierstoffsektor eine hohe wirtschaftliche Bedeutung.

Ester dieser Alkohole mit ungesättigten Fettsäuren, insbesondere die Ölsäureester, lassen sich auf klassisch chemischem Weg z.B. über saure Veresterung nur schwer herstellen. Eine enzymatische Herstellung dieser Ester aus Fettsäuren und Alkohol wird bisher aufgrund des hohen Enzymbedarfs nicht durchgeführt.

Aus der EP A-0-195 311 ist ein Verfahren zur Synthese von Triglyzeriden mittels immobilisierter Lipase bekannt. Dieses Verfahren ist zur Fettspaltung unter Verwendung und Recycling von Lipase kontinuierlich und semikontinuierlich geführt. Im Rahmen des Verfahrens erfolgt eine Zweiphasentrennung, wobei die mit Lipase angereicherte Zwischenschicht zusammen mit entweder der Ölphase (siehe Fig. 4) oder der wässrigen Phase (siehe Fig. 6) in einem Spaltreaktor rückgeführt wird.

Der Erfindung liegt die Aufgabe zugrunde, ein wirtschaftlich rentables enzymatisches Verfahren zur Herstellung von Fettsäuren sowie auch Fettsäureestern aus nativen Ölen und Fetten und eine entsprechende Vorrichtung anzugeben.

Diese Aufgabe wird durch den Gegenstand der unabhängigen Ansprüche gelöst. Bevorzugte Weiterbildungen sind in den Unteransprüchen definiert.

Die Erfinder haben ein wirtschaftlich effizientes enzymatisches Fett- bzw. Ölspaltungsverfahren und eine entsprechende Vorrichtung hierzu entwickelt und darüber hinaus festgestellt, daß sich beides in hervorragender Weise dazu verwenden läßt, die bisherigen Probleme bei der enzymatischen Spaltung und der enzymatischen Herstellung der in Frage stehenden Ester "auf einen Schlag" zu lösen.

Das erfindungsgemäße Verfahren zur enzymatischen Herstellung von Fettsäureestern zeichnet sich dadurch aus, daß man auf ein Gemisch aus einem ÖI bzw. Fett, Wasser und einem fett- bzw. öllöslichen Alkohol, insbesondere n- und/oder Iso-Alkoholen, als Biokatalysatoren für die Spaltung des Öls bzw. Fetts und die Esterbildung Lipasen einwirken läßt. Das hierbei gebildete Reaktionsgemisch wird zur Trennung der sich beim kombinierten Spalt- und Esterbildungsprozeß bildenden wässrigen, glycerinhaltigen Phase von der organischen Phase, die die Fettsäureester enthält, in eine selbstaustragende Zentrifuge gegeben. Diese wird so eingestellt, daß eine zwischen der wässrigen und organischen Phase gebildete, mit Lipase (Enzym) angereicherte Zwischenschicht sich in der Zentrifugentrommel ansammelt. Die Zentrifugentrommel wird zu vorgegebenen Zeitpunkten entleert und der dabei ausgetragene Zentrifugentommelinhalt wird in den kombinierten Spalt- und Esterbildungsprozeß zurückgeführt. Der Trommelinhalt kann auch für einen weiteren, anderen Spalt- und Esterbildungsprozeß ausgenutzt werden, indem er in diesen Prozeß eingetragen wird oder für einen späteren Prozeß lediglich bereitgestellt wird.

Ohne den Alkoholzusatz findet eine enzymatische Spaltung ohne gleichzeitige Esterbildung statt, wobei die Spaltung die äußerst wirtschaftliche Gewinnung von freien Fettsäuren und Glycerin ermöglicht. Ansonsten ist die grundsätzliche Verfahrensdurchführung identisch.

Als selbstaustragende Zentrifuge eignen sich die sogenannten selbstentschlammenden Separatoren, in deren Tellerpaket sich die besagte Zwischenschicht anreichern kann. Prinzipiell kann man jedoch auch in äquivalenter Weise anstelle des Tellerpaketes z.B. einen Rippeneinsatz oder andere interne Strukturen wie Flügel und dergleichen nutzen. Wichtig ist, daß die Zentrifuge selbstaustragend ist, so daß es von Zeit zu Zeit möglich ist, die in der Zentrifuge angesammelte Zwischenschicht durch eine Entleerung der Zentrifugentrommel auszutragen.

Die genannte Zwischenschicht bildet sich auch ohne den Alkoholzusatz bei der enzymatischen Fettspaltung, und zwar bei der Trennung der sich bildenden glycerinhaltigen wässrigen Phase und der die abgespaltenen freien Fettsäuren enthaltenden organischen Phase. Die Problematik dieser emulsionsartigen Zwischenschicht ist bekannt aus: "Continuous Use of Lipases in Fat Hydrolysis", M. Bühler and Chr. Wandrey, Fat Science Technology 89/ Dez. 87, Seiten 598 bis 605; "Enzymatische Fettspaltung", M. Bühler and Chr. Wandrey, Fett Wissenschaft Technologie 89 / Nr. 4 / 1987, Seiten 156 bis 164; und "Oleochemicals by Biochemical Reactions ?", M. Bühler and Chr. Wandrey, Fat Science Technology 94 / Nr. 3 / 1992, Seiten 82 bis 94.

In "Continuous Use of Lipases in Fat Hydrolysis" wird ÖI in einem ersten Rühr-Reaktor kontinuierlich gespalten. Das Reaktionsprodukt, das neben freien Fettsäuren Wasser, Glycerin und Mono- und Diglyceride, ungespaltenes ÖI sowie Enzym enthält, wird in eine Vollmantel-Tellerzentrifuge gegeben. Diese wird so eingestellt, daß die Zwischenschicht zwischen wässriger Glycerinphase und organischer Phase mit der organischen Phase ausgetragen wird. Die die Zwischenschicht enhaltende organische Phase wird einem zweiten Rühr-Reaktor zugeführt, dem ferner eine frische Wasser/ Enzymmischung zugeführt wird. Das Reaktionsprodukt des zweiten Reaktors wird wiederum einer Vollmantel-Tellerzentrifuge zugeführt, die allerdings nun so eingestellt wird, daß die Zwischenschicht mit der glycerinhaltigen wässrigen Phase und die entstehenden freien Fettsäuren ohne Emulsions-Zwischenschicht ausgeschleust werden. Die wässrige Phase wird in den ersten Reaktor zurückgeleitet, so daß dort die in der Emulsions-Zwischenschicht enthaltenen Enzymmengen dem Prozeß erneut zugeführt werden. Ohnehin werden darüber hinaus in der Regel bei selbstaustragenden Zentrifugen lediglich an der Trommelwand abgeschiedene Feststoffe diskontinuierlich mit den Trommelentleerungen ausgetragen

Auch bei der erfindungsgemäß durchgeführten Spaltung mit oder ohne gleichzeitiger Esterbildung tritt bei der Phasentrennung eine emulsionsartige Zwischenschicht auf, die neben der organischen Phase mit den gebildeten freien Fettsäuren bzw. deren Estern und der wässrigen glycerinhaltigen Phase beträchtliche Enzymmengen enthält. Durch eine außergewöhnlich betriebene Zentrifuge wird jedoch die Problematik erfolgreich gelöst und eine effiziente und nahezu verlustfreie Kreislaufführung vom Enzym bewerkstelligt. Hierdurch wird es möglich, bei hoher Enzymkonzentration und damit in kurzer Reaktionszeit ohne nennenswerte Enzymverluste die Fettspaltung mit hohem Spaltgrad und die Esterbildung mit hoher Ausbeute zu betreiben.

Es wird eine selbstaustragende Zentrifuge, bevorzugt ein sogenannter selbstaustragender Separator mit Tellerpaket, eingesetzt. Die Zentrifuge wird so eingestellt, daß weder die organische esterhaltige Phase noch die wässrige Phase nennenswerte Mengen enzymhaltiger Phasengrenzemulsion enthalten, sondern sich diese in der Zentrifuge, bevorzugt im Bereich der im Tellerpaket liegenden Trennzone eines Separators, anreichern. Dies stellt eine insofern ungewöhnliche Einstellung dar, als daß man auch bei den bekannten selbstaustragenden Separatoren, den sogenannten selbst entschlammenden Separatoren, üblicherweise gerade vermeidet, daß sich bei einer Flüssig/Flüssigphasentrennung größere Mengen einer sich bildenden Zwischenschicht im Tellerpaket ansammeln. Stattdessen sorgt man im allgemeinen dafür, daß diese Zwischenschicht möglichst gering ausfällt und in der nicht primär zu gewinnenden Phase mit ausgeschleust wird. So wurde im übrigen prinzipiell auch in der obigen Veröffentlichung vorgegangen, in der die Zwischenschicht hinter der zweiten kontinuierlich arbeitenden Mantelzentrifuge mit der wässrigen Phase ausgeschleust wurde und zum ersten Reaktor zurückgeleitet wurde. Darüber hinaus werden in der Regel bei selbstaustragenden Zentrifugen lediglich an der Trommelwand abgeschiedene Feststoffe diskontinuierlich mit den Trommelentleerungen ausgetragen.

Für den Fachmann bedeutet die erfindungsgemäße Einstellung der Zentrifuge, daß er durch die ihm geläufigen Maßnahmen der Einstellung der Wehre und/oder Einstellung des Gegendrucks im Ablauf der beiden zu trennenden flüssigen Phasen dafür sorgt, daß sowohl die organische Phase als auch die wässrige glycerinhaltige Phase möglichst klar und emulsionsfrei ablaufen.

Durch die Anwesenheit der Alkohole treten beim erfindungsgemäßen Verfahren gegenüber der reinen Spaltung weder ein nennenswert erhöhter Enzymbedarf noch eine erheblich verlängerte Reaktionszeit auf. Auch lassen sich die Ester auf einfache Weise aus dem Reaktionsgemisch gewinnen.

Die Alkohole werden in stöchiometrisch erforderlicher Menge für die Esterbildung, mit Vorteil jedoch in einem Überschuß von 2 bis 100 %, bevorzugt 5 % bis 20 %, gegenüber dem stöchiometrischen Bedarf den entsprechenden Ölen bzw. Fetten zugesetzt. Ein Überschuß an Alkohol beschleunigt dabei die Fettspaltung und bewirkt unerwarteter Weise in kurzer Zeit eine vollständige Spaltung aller Glyceride.

Das bei der Reaktion entstehende Glycerin geht aufgrund der äußerst guten Löslichkeit in Wasser und der sehr schlechten Löslichkeit in der hydrophoben organischen Phase in die Wasserphase über. Da die mittel- bis langkettigen Alkohole sehr schlecht wasserlöslich, jedoch sehr gut löslich in der organischen Phase sind, Wasser dagegen in der organischen Phase sehr schlecht löslich ist, werden sie entsprechend dem chemischen Gleichgewicht enzymatisch zu Fettsäureestern umgesetzt. Dies geschieht entweder durch Veresterung der aus der Fettspaltung stammenden Fettsäuren unter Abspaltung von Wasser, das in die wässrige Phase wandert, oder durch direkte Umesterung der Öle bzw. Fette unter Abspaltung von Glycerin, das ebenfalls in die wässrige Phase übergeht. Das chemische Gleichgewicht in der organischen Phase liegt somit ganz auf der Seite des Esters.

Durch einen überstöchiometrischen Zusatz von Alkohol kann das Gleichgewicht weiter auf die Seite des Esters verschoben und die Reaktionsgeschwindigkeit wesentlich erhöht werden. Schon bei einem geringfügig überstöchiometrischen Zusatz von Alkohol von etwa 5% ist die erfindungsgemäß durchgeführte enzymatische Fettspaltung in vielen Fällen nahezu vollständig. Dies wurde für eine Reihe von n- und Iso-Alkoholen von C8 bis C24 nachgewiesen. Die organische hydrophobe Phase enthält keine Mono-, Di- oder Triglyceride. Sie besteht lediglich aus Fettsäureestern des zugesetzten Alkohols, wenig freien Fettsäuren, der für deren Umsetzung erforderlichen Alkoholmenge und dem Alkoholüberschuß. Der Alkoholüberschuß einschließlich dieser noch nicht umgesetzten Alkoholmenge und die freien Fettsäuren lassen sich auf einfache Weise aus der organischen Phase entfernen und so die reinen Ester gewinnen.

Die Art der Maßnahmen zur Gewinnung der reinen Ester hängt von der Art der Fettsäuren und der Alkohole ab. Werden z.B. Ester von C18-Fettsäuren und C18-Alkoholen aus entsprechenden Ölen bzw. Fetten und Alkoholen erzeugt, lassen sich die freien C18-Fettsäuren zusammen mit dem C18-Alkoholüberschuß mittels Destillation aus dem Reaktionsendprodukt der Spaltung/Esterbildung entfernen, da die erzeugten Fettsäureester einen wesentlich niedrigeren Dampfdruck als die freien Fettsäuren und Alkohole haben.

Die destillativ abgetrennten Fettsäuren und nicht umgesetzter Alkohol können zur kombinierten Spalt/Esterbildungsreaktion zurückgeführt werden und so ebenfalls verlustfrei zu Estern umgesetzt werden. Bei kontinuierlicher Betriebsweise wird daher dem Ausgangsöl nur die stöchiometrische Menge zugesetzt, die Restmenge an Alkohol und die freien Fettsäuren werden kreislaufgeführt.

In einigen Fällen kann es vorkommen, daß die freien Fettsäuren und die entstehenden Ester etwa gleichen Dampfdruck aufweisen. Dies trifft z.B. bei Estern aus C18-Fettsäuren mit Iso-C13-Alkoholen zu. In diesen Fällen lassen sich die Überschußalkoholmengen des Iso-C13-Alkohols destillativ und die freien C18-Fettsäuren durch chemische Entsäuerung des als Sumpf bei der Destillation anfallenden Ester /Fettsäuregemisches abtrennen, freisetzen und rückführen.

Eine interessante Variante der Fettspaltung mit integrierter Ver- bzw. Umesterung ergibt sich bei Verwendung von Ölen, in denen die Fettsäuren nicht statistisch, sondern systematisch verteilt an den Alkoholgruppen des Glycerins gebunden sind wie z.B. die Erucasäure beim Crambeöl. Hier lassen sich bei geeigneter Wahl des spaltenden Enzyms gezielt nur Erucasäureester der zugesetzten Alkohole und Monoglyceride der anderen Fettsäuren des Crambeöls oder Erucasäurediglyceride und Fettsäureester der Nicht-Erucasäuren herstellen. Die Auftrennung der entstehenden Gemische kann dann in bekannter Weise vorzugsweise destillativ erfolgen. So läßt sich z.B. das bei kettenlängenspezifisch spaltenden Enzymen und Iso-C13-Alkohol als Alkoholkomponente entstehende Gemisch aus Diglycerid der Erucasäure, Iso-C13-Überschußalkohol und C18-Fettsäure-Iso-C13-Alkoholester auf einfache Weise destillativ trennen.

Von Vorteil kann es sein, das bei der enzymatischen Spaltung/Esterbildung entstehende Stoffgemisch, insbesondere nach Abtrennung einer selektiv durch ein spezifisches Enzym gebildeten Komponente (z.B. der C18-Fettsäure-Iso-C13-Alkoholester), in einem nachgeschalteten, zweiten enzymatischen Spaltprozess mit einem anderen Enzym umzusetzen und so die Fettsäuren bzw. Fettsäureester zu erhalten, für die dieses Enzym seine selektive katalytische Wirkung für den Spalt- und Esterbildungeprozeß entfaltet. So läßt sich ein weiter unten erläutertes von C18-Fettsäuren befreites Erucasäurediglycerid in einem zweiten enzymatischen Spaltprozess mit einem kettenlängen-unspezifischen Enzym zu Glycerin und Erucasäure bzw. in Anwesenheit von Alkohol zu Erucasäureester umsetzen.

Die erfindungsgemäße Durchführung der enzymatischen Fettspaltung gestattet somit, von Ölen und Fetten ausgehend, gezielt Fettsäuren und Fettsäureester herzustellen, die bisher nur wesentlich aufwendiger erzeugt werden konnten. Ausgenutzt werden hierbei die bekannten Wirkungen von Lipasen als Biokatalysatoren, die das chemische Gleichgewicht zwischen Estern, Alkoholen, Wasser und Säuren einstellen. Sie wirken insbesondere auf Fette und Öle. Letztere sind Glycerinfettsäureester, vor allem Triglyceride, mittel- bis langkettiger, in der Regel unverzweigter Fettsäuren. Lipasen arbeiten im Zweiphasensystem Öl bzw. Fettsäuren als hydrophobe Phase und Wasser als hydrophile Phase an der Phasengrenzschicht und stellen das chemische Gleichgewicht in den beiden Phasen ein. Die Lage des chemischen Gleichgewichts wird dabei von der Konzentration der jeweiligen Stoffe in den jeweiligen Phasen bestimmt.

Im Phasensystem Wasser/Öl bzw. native Fettsäure ist die Konzentration von Wasser in der Wasserphase dominierend, in der Ölphase dagegen sehr gering. Da Glycerin sehr gut in Wasser, kaum jedoch in der hydrophoben Phase aus Öl bzw. hydrophoben Fettsäuren löslich ist, muß im Gleichgewicht die Glycerinkonzentration in der Wasserphase wesentlich höher als in der Ölphase sein. In der Ölphase vorhandenes bzw. gebildetes Glycerin geht daher nahezu vollständig in die Wasserphase über. Native Fettsäuren mittlerer bis langer Fettsäureketten sind hydrophob und in Wasser nahezu unlöslich. Ihre Konzentration ist daher in der Wasserphase sehr niedrig. In der hydrophoben Phase sind sie dagegen sehr gut löslich, sie können sogar selbst die hydrophobe Phase darstellen. Wirken Lipasen an der Grenzschicht Wasser/ Öl auf das Öl, so wird dieses unter Verbrauch von Wasser über Diglyceride und Monoglyceride letztlich zu Fettsäuren und Glycerin gespalten. Im Gleichgewichtszustand gilt dabei nach dem Massenwirkungsgesetz für die beiden Phasen:

### Wasserphase

$$\{[\text{Fettsäuren}]^3 \times [\text{Glycerin}]\}/ \{[\text{Triglycerid}] \times [\text{Wasser}]^3\} = K$$

### Öl-/Fettsäurephase

$$\{[\text{Fettsäuren}]^3 \times [\text{Glycerin}]\}/ \{[\text{Triglycerid}] \times [\text{Wasser}]^3\} = K$$

Für das Reaktionsgleichungssystem der Triglyceridspaltung zu Fettsäuren gilt:

1.) Triglycerid + $H_2O$ ↔ Diglycerid + Fettsäuren
2.) Diglycerid + $H_2O$ ↔ Monoglycerid + Fettsäuren
3.) Monoglycerid + $H_2O$ ↔ Glycerin + Fettsäuren

Daraus resultiert nach dem Massenwirkungsgesetz:

$$k_1 = \{[Triglycerid] \times [H_2O]\}/\{[Diglycerid] \times [Fettsäuren]\}$$

$$k_2 = \{[Diglycerid] \times [H_2O]\}/\{[Monoglycerid] \times [Fettsäuren]\}$$

$$k_3 = \{[Monoglycerid] \times [H_2O]\}/\{[Glycerin] \times [Fettsäuren]\}$$

$$K = k_1 \times k_2 \times k_3 = \{[Triglycerid] \times [H_2O]^3\}/\{[Glycerin] \times [Fettsäuren]^3\}$$

In der organischen Phase ist die Wasserkonzentration $[H_2O]$ niedrig und konstant. Da Glycerin vorwiegend in der Wasserphase in Lösung geht, wird ebenfalls die Glycerinkonzentration [Glycerin] in der organischen Phase niedrig und damit quasi konstant. Damit ergibt sich:

$$K \times [Glycerin]/[H_2O]^3 = K' = [Triglycerid]/[Fettsäuren]^3 .$$

Aufgrund der Unterschiede in der Löslichkeit der jeweiligen Komponenten in der hydrophilen und hydrophoben Phase spalten Lipasen bei hoher Konzentration von Wasser in der hydrophilen Phase Fette und Öle nahezu vollständig in Glycerin und Fettsäuren. Das gebildete Glycerin löst sich dabei im Wasser, die Fettsäuren zunächst im Öl, bis sie zuletzt eine eigene, hydrophobe Fettsäurephase bilden.

Wird die enzymatische Fettspaltung erfindungsgemäß in Anwesenheit zusätzlicher, anderer Alkohole als dem in Fetten und Ölen enthaltenen Alkohol Glycerin durchgeführt, so stellen auch hier die Lipasen das chemische Gleichgewicht in der hyrophilen und hydrophoben Phase ein. (Ausgenommen hiervon sind Alkohole und Alkoholkonzentrationen, die die Wirksamkeit des Enzyms beeinträchtigen bzw. mit dem Enzym unverträglich sind und es deaktivieren.) Auch in diesem Fall wird die Lage des chemischen Gleichgewichts vom Verteilungskoeffizienten der jeweiligen Komponenten zwischen der hydrophilen und hydrophoben Phase bestimmt. Die Berechnung bzw. die Abschätzung der chemischen Gleichgewichtsverteilung in den beiden Phasen ist naturgemäß komplizierter als bei der einfachen Fett- bzw. Ölspaltung. Dies gilt insbesondere für mehrwertige Alkohole, die wasserlöslich sind wie z.B. Trismethylolpropan und Pentaerythrit.

Wesentlich einfacher zu kalkulieren ist das Verhalten von hydrophoben in Wasser praktisch unlöslichen Alkoholen (Mono- wie auch Polyole). Zu derartigen Alkoholen zählen die erfindungsgemäß eingesetzten mittel- (ab C6-Kettenlänge) bis langkettigen, n- und Iso-Alkohole. Diese Alkohole sind dem Zweiphasensystem zugesetzt praktisch nur in der organischen Phase löslich. Die wässrige glycerinhaltige Phase enthält entsprechend der geringen Sättigungskonzentration nur sehr wenig Alkohol. Ein Zusatz von solchen Alkoholen verschiebt das Spaltgleichgewicht durch Esterbildung und Verbrauch an freien Fettsäuren in Richtung Spaltung. Dies wird verstärkt durch Zugabe eines Alkoholüberschusses, so daß im Gleichgewicht in der organischen Phase keine Tri-, Di- und Monoglyceride mehr sein können. Für derartige gut in der organischen Phase lösliche Alkohole kann man die obige Reaktion in Anwesenheit von Alkohol wie folgt beschreiben:

Grundsätzlich gilt unabhängig von der Art des Alkohols:

$$Ester + H_2O \leftrightarrow Fettsäuren + Alkohol$$

$$k_{Ester} = \{[Ester] \times [H_2O]\}/\{[Fettsäuren] \times [Alkohol]\}$$

Anders als bei wasserlöslichen Alkoholen gilt für gut in der organischen Phase lösliche Alkohole: [Alkohol] ist in $H_2O$ gesättigt und damit konstant. Damit gilt:

$$K' \times k_{Ester} / [H_2O] = K'' = \{ [Triglycerid] \times [Ester]\}/\{[Fettsäuren]^4 \times [Alkohol]\}$$

Das erfindungsgemäße Verfahren läßt sich mit gutem Erfolg mit Alkoholen durchführen, deren Löslichkeit in Wasser kleiner 5 Gew.% bezogen auf die wässrige Phase ist. Die Ausbeute und Umsetzungsrate nehmen bei steigender Wasserlöslichkeit des Alkohols ab. So wurde z.B. TMP als Alkohol im erfindungsgemäßen Verfahren eingesetzt, woraufhin die Umsetzungsrate um etwa 50% sank.

Einige Lipasen, sogenannte spezifische Lipasen, sind nicht fähig, sämtliche glyceridische Fettsäureesterbindungen zu spalten. Insbesondere die mittlere, am C2 des Glycerins gebundene Fettsäure kann von bestimmten Lipasen nicht abgespalten werden. Durch Verwendung derartiger Lipasen können daher gezielt z.B. Monoglyceride und Fettsäuren erzeugt werden. Enthalten die Ausgangsöle bestimmte Fettsäuren nicht statistisch, sondern systematisch verteilt im Öl oder Fett glyceridisch gebunden, so können mit spezifisch wirkenden Lipasen Fettsäuren bzw. deren Ester gewonnen werden, die nicht dem im Triglycerid vorhandenen Fettsäuremuster entsprechen.

So ist bekannt, daß langkettige Fettsäuren mit Kettenlängen > 20 wie Erucasäure in nativen Ölen und Fetten stets an den äußeren Hydroxygruppen des Glycerins, nicht jedoch an der mittleren Hydroxygruppe gebunden vorliegen. Bei Ölen wie z.B. erucasäurereichem Crambeöl mit über 60 Gew.% Erucasäure ( und ca. 6 Gew.% Fettsäuren > C22 ) sind praktisch alle Fettsäuren mit Kettenlängen > C20 am C1 und C3 des Glycerins gebunden, die restlichen ca. 33,33 Mol. % C18-Fettsäuren sind am C2 des Glycerins gebunden. Mit einer spezifischen Lipase kann man nun gezielt nur die endständigen Säuren abspalten und deren Ester erzeugen. Die Erucasäureester können dann z.B. über fraktionierte Kristallisation isoliert werden. Auch in diesem Fall wirkt das Enzym an der Phasengrenzschicht. Es kann daher erfindungsgemäß effizient kreislaufgeführt werden.

Weiterhin ist bekannt, das die Wirksamkeit von Lipasen oftmals, je nach Typ der Lipase, abhängig von der Kettenlänge der jeweiligen Fettsäuren oder vom Sättigungsgrad und von der Konformation (Cis, Trans, konjugiert oder nicht konjugiert usw.) der Fettsäuren ist. Das erfindungsgemäße Verfahren kann auch diese Effekte zur gezielten Erzeugung von Fettsäuren bzw. deren Estern nutzen.

Auch in diesem Fall findet die Enzymkatalyse in der Grenzschicht der hydrophilen und der hydrophoben Phase statt. Die Kreislaufführung des Enzyms und Ausschleusung von Komponenten ermöglichen dabei die gezielte Erzeugung gewünschte Produkte. Dies kann beispielhaft wiederum am oben erwähnten Crambeöl erklärt werden.

Die unspezifische Lipase aus Candida Rugosa spaltet langkettige Fettsäuren wie Erucasäure wesentlich langsamer als C16 und C18 Fettsäuren. Erfindungsgemäß durchgeführt, führt die enzymatische Fettspaltung mit Lipase aus Candida Rugosa zu Erucasäure-1,3-Diglycerid und den am C2 des Glycerins abgespaltenen Fettsäuren. Letzteres sind C18-Fettsäuren. Mittels einer Kurzwegdestille lassen sich diese Fettsäuren und auch deren Ester aus dem Diglycerid in vorteilhafter Weise als Destillat abtrennen. Analoge Fälle lassen sich für eine Vielzahl anderer Fettsäuren wie z.B. Omega-3- und Omega-6-Fettsäuren bei Verwendung geeigneter Enzyme realisieren.

Wie bereits oben erwähnt, wird zur Abtrennung der Fettsäuren aus dem Reaktionsprodukt der Spaltreaktion oder alternativ der Fettsäureester aus dem Reaktionsprodukt der Spalt/Esterbildungsreaktion vorzugsweise die Vakuumdestillation angewandt, und zwar bevorzugt eine schonende Kurzwegdestillation. Bei erzeugten Fettsäureestern mit niedrigerem Dampfdruck als dem der freien Fettsäuren und Alkohole enthält das Destillat die Überschußmenge an n- oder Iso-Alkohol, nicht umgesetzte Alkoholmengen und freie Fettsäuren. Das Destillat wird bevorzugt in den Spalt/Esterbildungsprozeß zurückgeführt.

Alternativ zur destillativen Abtrennung der Fettsäureester bzw. Fettsäuren kann eine adsorptive Trennung (z.B. Säulenchromatographie) eingesetzt werden.

Vakuum-Dünnfilm-Verdampfer wie Fallfilmverdampfer oder Kurzwegdestillen sind im Vergleich zur Blasendestillationstechnik schonender und vor allem kontinuierlich betreibbar, weshalb sie bevorzugt eingesetzt werden. Zudem verlangen diese Destillationstechniken ohnehin einen flüssigen Restrückstand von mindestens ca. 5% bis 10%, da andernfalls der Destillationsfilm reißt. Diese Bedingung wird erfindungsgemäß sowohl bei er Spaltung als auch beim kombinierten Spalt/Esterbildungsprozeß eingehalten.

Der aus der Destille ablaufende Sumpf enthält, beim kombinierten Spalt/Esterbildungsprozeß, wie weiter oben bereits ausgeführt, entweder die gewünschten Ester oder die Ester und noch nicht umgesetzte freie Fettsäuren. Im Falle der reinen Spaltreaktion enthält das Destillat die abgespaltenen Fettsäuren. Der Sumpf bzw. Destillenrückstand enthält die nicht umgesetzten Triglyceride und wird in den Spaltprozeß zurückgeführt.

Die erfindungsgemäß in der Zentrifuge, nach der bevorzugten Ausführung im Tellerpaket eines Separators, angesammelte emulsionsartige Zwischenschicht wird diskontinuierlich durch periodische Vollentleerungen der Trommel ausgetragen und die ausgetragene, Enzym enthaltende Zwischenschicht wird wiederverwendet. Dabei ist es zweckmäßig, die Trommelentleerung immer dann vorzunehmen, wenn die ausgeschleuste organische Phase und/oder die wässrige glycerinhaltige Phase gerade noch nicht beginnen, sich mit ausgeschleuster Zwischenschicht einzutrüben. Häufigere Entleerungen zu vorgegebenen Zeitpunkten sind ebenfalls möglich, werden jedoch nicht bevorzugt. Die Tatsache, daß bei der Vollentleerung auch wässrige Phase und organische Phase mit ausgetragen werden, ist nicht nachteilig, da sämtliche Phasen durch Rückführung in den Reaktor wiederverwendet werden. Statt einer Vollentleerung sind auch Teilentleerungen anwendbar, die allerdings so einzustellen sind, daß die Zwischenschicht möglichst vollständig ausgeschleust wird.

Auf die dargelegte Weise lassen sich die Verluste an mit der wässrigen Phase und organischen Phase ausgetragenem Enzym drastisch senken. Im Vergleich zum zeitabhängigen Enzymverbrauch (Enzymalterung) sind die Ausschleusungsverluste bei dieser erfindungsgemäßen Verfahrensweise sehr gering.

Eine technisch interessante Ergänzung der Erfindung zur weiteren Herabsetzung eines Enzymverlustes in der abgetrennten organischen Phase besteht in der Verwendung eines zusätzlichen selbstaustragenden Polierseparators. Dieser zusätzliche Separator wird unmittelbar hinter dem Separator der einzigen oder letzten Spalt- bzw. Spalt/Esterbildungsstufe vorgesehen und erhält von diesem die ausgeschleuste organische Phase. Es handelt sich bei diesem zusätzlichen Separator vorzugsweise um eine selbstaustragende Zentrifuge mit Tellerpaket, die so eingestellt wird, daß sich die sedimentierenden Feststoffe, d.h. hier die Enzyme, und die noch abschleuderbaren Reste an wässriger Phase an der Trommelwand abscheiden. Die so abgeschleuderten Enzymmengen werden dann wiederum diskontinuierlich ausgetragen und in den Spalt- bzw. Spalt/Esterbildungsprozeß zurückgeführt.

Vorteilhaft läßt sich vor allem bei Verwendung einer selbstaustragenden Zentrifuge zur Abtrennung der fettsäureesterhaltigen organischen Phase die Spaltreaktion in Schlaufenreaktoren diskontinuierlich, oder mit anderen Worten, absatz- oder batchweise durchführen und nicht kontinuierlich und in Durchlaufreaktoren. So wird z.B. ein Reaktor mit Öl befüllt, wobei dessen Schlaufe für die Umwälzung des Reaktorinhalts aus Öl bzw. Fett, Wasser, Alkohol und Enzym nicht aktiv ist.

Ein weiterer Reaktor führt gleichzeitig die Spalt- bzw. Spalt/Esterbildungsreaktion durch mit aktiver Schlaufe und ein dritter Reaktor wird während dieser Zeit über einen selbstaustragenden Separator abgefahren, wobei das Reaktionsgemisch in wässrige Glycerin haltige Phase, organische Phase mit abgetrennten Fettsäuren bzw. Fettsäureestern und enzymhaltige Emulsionsgrenzphase, die sich als Zwischenschicht bildet, getrennt wird. Die Emulsionsgrenzphase wird von Zeit zu Zeit diskontinuierlich aus dem Separator ausgetragen, durch Versetzen mit frischem Enzym nachgeschärft und dem Reaktor wieder zugeführt.

Die Reaktion kann so auf sehr hohem Enzymniveau gefahren werden, da durch die Umwälzung und die in den Umwälzpumpen zur Wirkung kommenden Scherfelder des Schlaufenreaktorbetriebs eine außerordentlich große Phasengrenzfläche entsteht. Dabei ist es möglich, die zugeführte Wassermenge gering zu halten und eine wässrige Phase mit wesentlich erhöhter Glycerinkonzentration zu gewinnen, als dies zuvor in den erwähnten Spaltprozessen möglich war. Selbst bei über 30 Gew.% Glycerin in der ausgeschleusten wässrigen Phase findet in diesem Fall keine nennenswerte Verlängerung der Reaktionszeit statt. Derartig hohe Glycerinkonzentrationen hatte man bislang nicht für praktikabel gehalten. Weiterhin lassen sich durch die hohe Glycerinkonzentration die Enzymverluste drastisch senken.

Somit kann erfindungsgemäß die Wasserzugabe sowohl beim reinen Spaltprozeß als auch beim kombinierten Spalt/Esterbildungsprozeß vorteilhaft gering sein. Bezogen auf die eingesetzte organische Phase aus eingesetztem Öl oder Fett und Alkohol wird bei letzterem mindestens 5 Gew.% Wasser zugesetzt. Die Zugabe von mehr als 200 Gew.% Wasser ist möglich, erschwert jedoch nur unnötig den gesamten Prozeß. Wenn man zudem den obigen Vorteil einer hohen durch die Erfindung möglichen Glycerinkonzentration im Bereich von etwa 10 bis 35 Gew.% bezogen auf die entstehende glycerinhaltige wässrige Phase nutzen will, arbeitet man bevorzugt im Bereich einer Wasserzugabe von 20 bis 30, maximal 50 Gew.% bezogen auf die eingesetzte organische Phase. Auch beim reinen Spaltprozeß arbeitet man im Bereich einer Wasserzugabe von 5 bis 200 Gew.%, bevorzugt 20 bis 30, maximal 50 Gew.% bezogen auf die eingesetzte organische Phase.

Erfindungsgemäß kann der Prozeß auf hohem Enzymniveau gefahren werden, ohne jedoch viel Enzym zu verbrauchen. So wird erfindungsgemäß die Lipase auch bei hoher Enzymaktivität in der Regel mit mindestens 0,01 Gew.% bezogen auf das eingesetzte Öl oder Fett als im Reaktor wirksame Menge vorgelegt. Ein derzeit bevorzugter Bereich für die vorgelegte Lipasemenge liegt bei den in den Ausführungsbeispielen angeführten Lipasen zwischen 0,1 und 0,5 Gew.% bezogen auf das eingesetzte Öl oder Fett. Dieses hohe Enzymniviau beschleunigt den Spalt- und auch den Spalt/Esterbildungsprozeß ungemein. Andererseits ist durch die Enzymrückführung bedingt, der tatsächliche Enzymverbrauch sehr gering, so daß nur Bruchteile der vorgelegten Mengen nachdosiert werden müssen. In den Versuchen betrugen die nach zu dosierenden Lipasemengen weniger als zehn Prozent von der vorgelegten, im Reaktor wirksamen Menge. Der Fachmann weiß, daß er das optimale Enzymniveau nicht nur enzymtypspezifisch, sondern auch in Abhängigkeit von der Enzymaktivität des jeweiligen Präparates auswählen muß. Schließlich ist zu beachten, daß beim Übergang auf ein immer geringeres Enzymniveau der Prozeß auch immer langsamer wird. Zudem ist bekannt, daß die Steigerung des Enzymniveaus über bestimmte Werte hinaus keinen verfahrenstechnischen bzw. wirtschaftlichen Vorteil mehr bringt.

Dies ist auch bereits in den weiter oben erwähnten Veröffentlichungen dargelegt worden. Der Fachmann kann unter Berücksichtigung dieser Tatsachen, für die jeweiligen Ausgangskomponenten seines Prozesses das optimale Enzymniveau mit wenigen Versuchen bestimmen.

Erfindungsgemäß kann in Anwesenheit von Alkohol die Spalt- bzw. Spalt/Esterbildungsreaktion mit vollständiger Freisetzung des Glycerins in nur einer Stufe durchgeführt werden, wobei in dieser Stufe das Enzym im Kreislauf geführt wird.

Die erfindungsgemäße Spalt- bzw. Spalt/Esterbildungsreaktion wird jedoch bevorzugt z. B. mit Umwälzreaktoren mehrstufig, beispielsweise in zwei Stufen durchgeführt. Die in der zweiten Stufe gewonnene wässrige Glycerinphase wird dann der ersten Stufe als Wasserphase zugeführt und der zweiten Stufe wird als Wasserphase Frischwasser und ferner die in der ersten Stufe gewonnene organische Phase zugeführt. Die zwei- oder mehrstufige Reaktion ist von Vorteil, weil die Enzymverluste so weiter minimiert werden können.

Das erfindungsgemäße Verfahren ist auch dazu geeignet, die Mono-, Di- und Triglyceride aus sogenannten Soap-Stocks aus der Alkali-Raffination von Speiseölen zu spalten und zu Fettsäureestern umzusetzen. Hierzu setzt man vorzugsweise vor der Spaltung/Esterbildung durch Zugabe von Säure die in den Seifen gebundenen Fettsäuren frei.

Die Erfindung wird nun anhand von Ausführungsbeispielen und der beiliegenden Zeichungen erläutert, wobei

FIG.1a eine schematische Darstellung eines Beispiels für einen industriellen Prozeß nach der Erfindung zeigt, der für den Fall ausgelegt ist, daß die entstehenden Fettsäureester destillativ von den noch nicht umgesetzten Fettsäuren und dem Alkohol abtrennbar sind,

FIG.1b eine entsprechende schematische Darstellung eines modifizierten Prozesses für den Fall zeigt, daß die Fettsäureester nur zusammen mit den freien Fettsäuren destillativ abgetrennt werden können, und

FIG. 2 eine schematische Darstellung eines Beispiels für einen industriellen Spaltprozesses nach der Erfindung zeigt, wobei keine Veresterung stattfindet und ohne Zugabe von Alkohol gearbeitet wird.

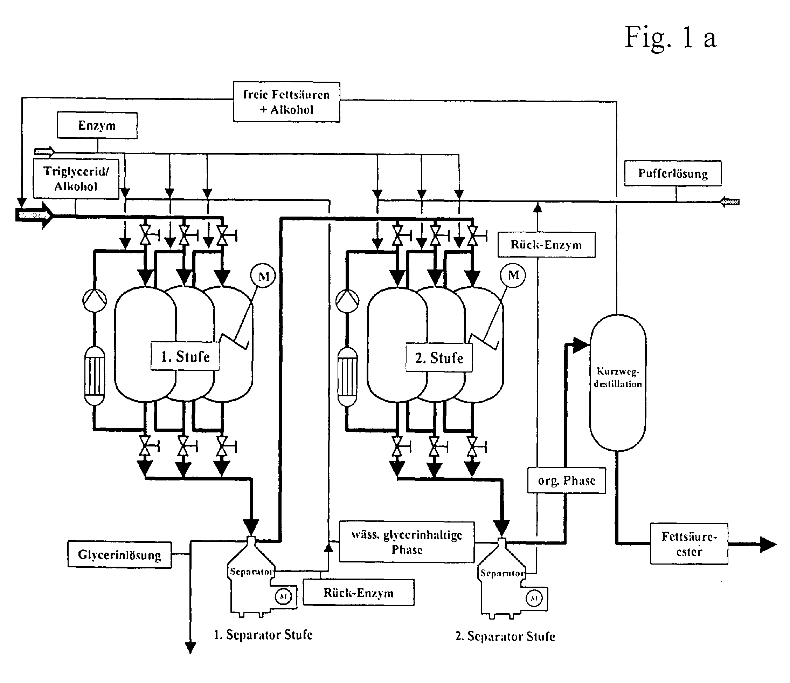

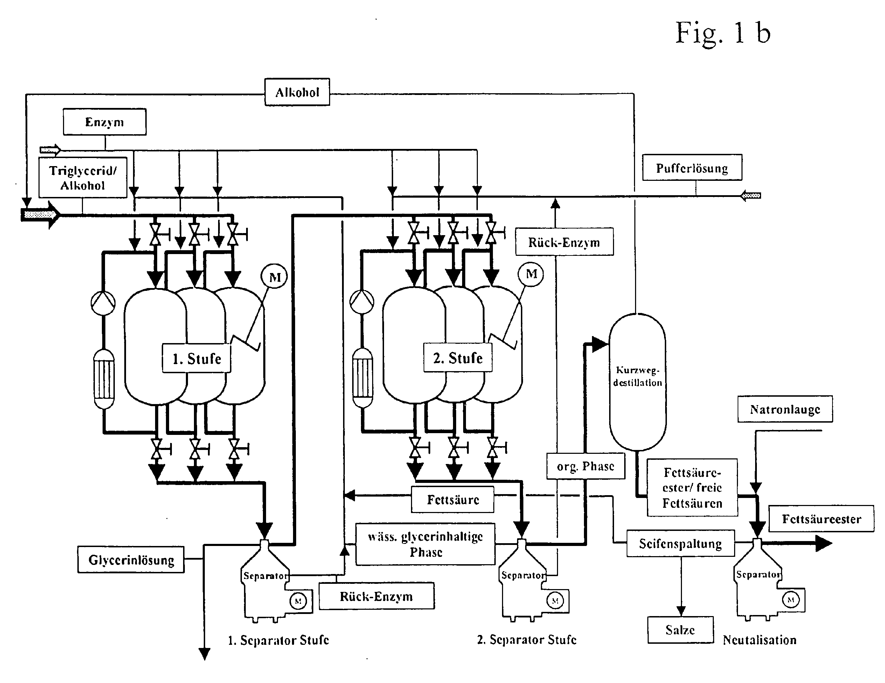

Die FIG.1a und 1b zeigen eine erfindungsgemäße Anordnung zur zweistufigen kombinierten Fettspaltung/ Festtsäureesterbildung, die dazu geeignet ist, die aufgezeigten Merkmale im Produktionsmaßstab zu realisieren. Es sind zwei Prozeßstufen 1 und 2 vorgesehen, die jeweils drei Reaktoren mit Umwälzungsschlaufe umfassen. Die Reaktoren werden, wie weiter oben bereits dargelegt, intermittierend betrieben: Befüllen, Reaktionsphase, Entleerungsphase. Die jeweils für jeden der drei Reaktoren einer Stufe vorgesehene Umwälzungsschlaufe ist mit einer in der Zeichnung angedeuteten Kreiselpumpe und mit einem vorgeschalteten Wärmeaustauscher realisiert. Die Reaktoren bestehen z. P. aus Edelstahlbehältern mit Rührwerk. Ferner ist für jede Stufe ein Separator in Form eines selbstaustragenden Teller-Separators vorgesehen.

Der Ausgang des Separators der zweiten Spaltstufe, aus dem die organische Phase mit den Fettsäureestern ausgeschleust wird, ist mit einer Vakuum-Kurzweg-Destille verbunden, in der eine Kurzwegdestillation zur Abtrennung der freien Fettsäuren und des Alkohols von den gebildeten Fettsäureestern erfolgt - für den oben dargelegten Fall, daß die letzteren einen niedrigeren Dampfdruck haben als die ersteren.

Das Rückstandsprodukt der Destillation enthält bereits die gewünschten Ester, wenn die noch nicht umgesetzten freien Fettsäuren und der Alkohol leichter flüchtig als das Endprodukt in Form der gewünschten Fettsäureester sind. Dies entspricht dem in Fig.1a skizzierten Prozeß.

Wird gemäß Fig.1b stattdessen als Destillat nur der Alkohol gewonnen, enthält das Rückstandsprodukt der Destille sowohl die Ester als auch freie Fettsäuren. In einem Separator werden die freien Fettsäuren durch Zugabe einer alkalischen Lösung, z.B. Natronlauge, neutralisiert und können dann als schwerere Seifenphase von den Fettsäureestern zentrifugal abgetrennt werden. Die Seifenphase wird in an sich bekannter Weise z.B. in einer weiteren Zentrifuge unter vorheriger Zugabe von Säure, z.B. Schwefelsäure, in Fettsäuren und Salze gespalten, wobei die Fettsäuren in die erste Stufe der Reaktion zurückgeleitet werden.

Den Reaktoren werden eine Pufferlösung, das zu spaltende Triglycerid, der jeweilige esterbildende Alkohol und Enzym, d.h. Lipase, zugeführt. Ferner wird Enzym bei jeder intermittierend herbeigeführten Entleerung der Separatoren gewonnen und zurück in den gerade zu befüllenden Reaktor derselben Stufe geleitet, dem auch der jeweilige Separator zugeordnet ist. Somit bleibt das Enzym mit gleichzeitig ausgeschleusten Anteilen an freien Fettsäuren, noch nicht gespalten Triglyceriden usw. im Kreislauf einer Stufe. Hierdurch ist verhindert, daß sich die Ausgangsprodukte unterschiedlicher Qualität der beiden Stufen wieder teilweise vermischen. Auch die Gefahr von Rückreaktionen ist hierdurch verringert. Schließlich sei noch erwähnt, daß dem Separator der ersten Stufe GlycerinLösung als die abgetrennte schwerere flüssige Phase entnommen wird und zur Weiterverarbeitung bereit steht. Die Glycerinlösung aus dem Separator der zweiten Stufe wird gemäß den Figuren dem zu befüllenden Reaktor der ersten Stufe zugeführt.

Als Pufferlösung wird eine leicht saure Standardlösung, die nach den Maßgaben des Enzymherstellers ausgewählt und eingestellt wird, eingesetzt. In den Ausführungsbeispielen wurde eine leicht sauer gestellte wässrige Lösung mit Natriumacetat und Essigsäure eingesetzt. Der optimale PH-Wert der Pufferlösung wird nach dem jeweiligen Enzym eingestellt.

Dies gilt auch für die Temperatur in der Prozeßführung. In den Versuchen wurden Temperaturen zwischen 25 und 45 °C getestet. Hierbei ist zu beachten, daß die Temperatur aufgrund der exothermen Reaktion ohnehin leicht erhöht ist. In den getesteten Fällen war es jedoch ohne weiteres möglich, dafür zu sorgen, daß die wässrige und organische Phase flüssig gehalten wurden und Kristallisationen in den flüssigen Phasen nicht auftraten.

Als Enzyme kommen prinzipiell alle Enzymsorten in Betracht, die in den eingangs erwähnten Veröffentlichungen von Bühler und Wandrey dargelegt sind. Es wurden verschiedene Candida Rugosa Enzyme gründlich erprobt. Darüber hinaus eignetsich das erfindungsgemäße Verfahren auch für die Verwendung von aus Ölsaaten (z.B. aus Ricinusöl) gewinnbaren Enzymen. Diese haben naturgemäß den Vorteil einer besonderen selektiven Wirksamkeit für bestimmte Fettsäuren. Sie werden jedoch bisher kaum zur Fettspaltung eingesetzt und sind in der Regel teurer als andere fermentativ industriell aus Hefen, Pilzen, Bakterien und dergleichen hergestellte Enzyme.

In den Versuchsreihen wurden als n-Alkohole Oleylalkohol und Stearylalkohol erprobt. Als Iso-Alkohole wurden Versuchsreihen mit Iso-C8, Iso-C10, Iso-C13, Iso-C16, Iso-C18, Iso-C20 und Iso-C24 gefahren.

Die Ester wurden aus High-Oleic-Sonnenblumenöl hergestellt. Je nach Anwendungsfall kann man von entwachstem und raffiniertem oder auch rohem Öl oder Fett ausgehen.

Es ist auch möglich, die Erfindung mit weniger gängigen und speziell längerkettigen Alkoholen über C26 bis zu C36 und mit anderen Ölen und Fetten auszuführen.

Beispiele

1. Enzymatische Esterbildung

Ein Ansatz von etwa 20 kg entwachstem und raffiniertem High Oleic Sonnenblumenöl 90plus® wurde in einem Rührgefäß (80 I Volumen) vorgelegt, mit 22,3 kg (10% stöchiometrischem Überschuß) Isofol 20 (C-20 Alkohol der Fa. Fuchs Petrolub), 10,6 kg Pufferlösung (0,1 n Na- Azetat/ Essigsäure, pH 4,6) und 40 g OF-Enzym 360 (Fa. Meito Sangyo) vermischt und 3 Stunden bei etwa 40°C mittels Kreiselpumpe umgepumpt.

Danach wurde die Mischung im freien Gefälle bei einer Leistung von etwa 30 kg/h Phasensumme direkt in eine Tellerzentrifuge (SA 1-01, Westfalia Separator AG, Oelde) geführt und kontinuierlich getrennt. Die Säurezahl der ablaufenden organischen Phase wurde mittels Titration (in alkoholischer Lösung mit 0,1 n KOH nach DIN 53169 und DIN 53402) bestimmt und durchlief ein Maximum bei etwa 55, um gegen Ende auf etwa 15 abzufallen.

Das Enzym wurde zusammen mit geringen Mengen an organischer Phase und Glycerinwasser aus der Zentrifuge ausgeschleust. Es zeigte kaum Aktivitätsverlust und konnte wieder verwendet werden.

Aus der Zentrifuge konnten eine klare Öl-/Esterphase und eine klare Glycerinlösung als wässrige Phase abgefahren werden. Die wässrige Phase enthielt die erwartete Menge an Glycerin als ca. 17 Gew.%-ige Lösung.

In der Öl-/Esterphase konnte mittels Dünnschichtchromatographie kein Tri-, Di-, oder Monoglycerid nachgewiesen werden. Nach einer abschließenden Vakuumdestillation eines Aliquots von 4 kg wurde der entsprechende C-20 Iso- Ester als Rückstand in 95 %-iger Ausbeute bezogen auf die eingesetzte Ölmenge erhalten.

Das Destillat enthielt die Überschußmenge des Iso-Alkohols und die restlichen nicht umgesetzten Mengen an Iso-Alkohol und freien Fettsäuren.

Ein Teil des Destillats wurde im Laborversuch einem stöchiometrischen Ansatz aus Öl , Iso-Alkohol und Pufferlösung zugesetzt, so daß der Alkoholüberschuß bezogen auf die neu eingesetzte Ölmenge wieder bei 10% lag.

Die Ausbeute erreichte in gleicher Zeit bei gleicher Menge an Enzym ebenfalls 95 %. Auch hier konnten nach Abschluß der Reaktion aus der abzentrifugierten organischen Phase vergleichbare Mengen an nicht umgesetztem Iso-Alkohol und freien Fettsäuren abdestilliert werden, so daß eine verlustfreie Kreislaufführung des Destillats möglich war. Es ist damit auch bewiesen, daß das eingesetzte Enzym (OF 360) die Veresterung von freien Fettsäuren und Iso- Alkohol in Anwesenheit einer wässrigen Phase katalysiert.

Die Wirksamkeit des erfindungsgemäßen Verfahrens wurde ferner für die n-Alkohole Oleylalkohol und Stearylalkohol erprobt. So wurde z.B. ein Versuch mit einem Oleylalkohol (MG= 268,49) und der im obigen Beispiel eingesetzten Lipase mit gleichem Erfolg durchgeführt. Als Iso-Alkohole wurden Iso-C8, Iso-C10, Iso-C13, Iso-C16, Iso-C18, Iso-C20 und Iso-C24 eingesetzt. Darüber hinaus wurden auch erfolgreiche Versuche mit Crambeöl gemacht, wobei sich die weiter oben dargelegten Möglichkeiten zeigten. Ein verzweigtes C16/C18 Fettalkoholgemisch (MG=286) konnte ebenfalls mit der obigen Lipase erfindungsgemäß erfolgreich umgesetzt werden.

Prinzipiell kann das erfindungsgemäße Verfahren auch auf synthetische Fettsäureester, z.B. synthetische Triglyceride und andere Polyolester angewandt werden.

2. Enzymatische Spaltung

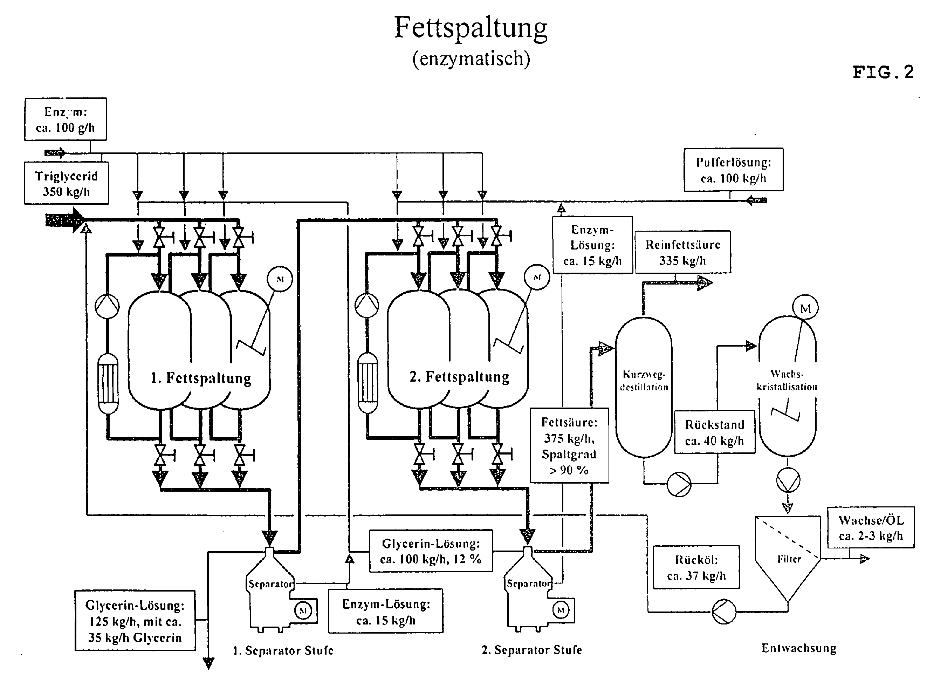

Die FIG.2 zeigt deutlich auf, daß sich die Prozeßführung für die reine Fettspaltung ohne gleichzeitige Esterbildung durch Alkoholzusatz in wesentlichen Punkten nicht von dem oben bereits u.a. anhand der FIG. 1a und 1 b gezeigten Verfahrensführung unterscheidet. Es sind beispielhalber mögliche Mengendurchsätze angegeben, jedoch kann und wurde der Prozeß auch mit anderen Mengen erfolgreich gefahren. Es wird daher auch nur der sich unterscheidende Teil des Prozesses erläutert. Die obigen Ausagen für mögliche Temperaturbereiche und Handhabung der Enzyme treffen gleichermaßen zu.

So ist der Ausgang des Separators der zweiten Spaltstufe, aus dem die organische Phase mit den Fettsäuren (statt der Fettsäureester) ausgeschleust wird, wiederum mit einer Vakuum-Kurzweg-Destille verbunden, in der eine Kurzwegdestillation zur Abtrennung der freien Fettsäuren erfolgt. Das Rückstandsprodukt der Destillation wird einem Kristallisator mit Rührwerk zugeführt, in dem eine Wachskristallisation erfolgt. Im Anschluß daran wird das Rückstandsöl, in dem Wachse und andere höher siedende Begleitstoffe als Feststoffe auskristallisiert sind, über eine Pumpe in eine Filtereinrichtung eingebracht und dort von diesen Begleitstoffen befreit. Das so gereinigte Öl wird dann zur Spaltung vor die erste Stufe zurückgeleitet.

Eine Spaltung erfolgte mit High-Oleic Sonnenblumenöl. Nach einer Startphase wurden in einem Reaktor der ersten Stufe 30.0 kg rohes, unraffiniertes High-Oleic Sonnenblumenöl 90 plus (eingetragenes Warenzeichen) der Firma Dr. Frische GmbH, (mit einer Säurezahl von 4, ermittelt durch Titration gegen alkalische Kalilauge nach DIN 53169 und DIN 53402), zusammen mit 7.0 kg einer Pufferlösung vorgelegt, die aus einer mit 3.0 g Natriumacetat gepufferten 12 Gew.%igen Glycerin-in-Wasser-Lösung bestand. Die zugeführte Mischung aus Öl und Pufferlösung wurde unter Rühren mit der Kreiselpumpe umgewälzt und auf 35-40°C temperiert. Danach wurden 2 kg mit Enzym angereichertes Entleerungprodukt aus der ersten Separatorstufe der Startphase eingesetzt und 3g frische Lipase des Enzyms aus "candida rugosa" (Lipase in Form eines gepulverten Feststoffes von Meito Sangyo, Japan, 360000 U/g ) zugegeben, das auch in der Startphase verwendet wurde. Nach 60 Minuten Reaktionszeit unter Rühren wurde die anschließend beruhigte Reaktionsmischung im Separator der ersten Stufe (selbstaustragende Teller-Zentrifuge SA1-01 der Firma Westfalia Separator AG) in eine organische, fettsäurehaltige Phase und eine wässrige Glycerin-Phase getrennt, wobei ca. 40 kg/h Reaktorinhalt über die Zentrifuge flossen und die Trommel der Zentrifuge alle 15 Minuten hydraulisch vollständig entleert wurde. Das Entleerungsprodukt der Trommel wurde dem jeweils gerade zu befüllenden Reaktor der ersten Stufe zugeführt. Der zu befüllende Reaktor der zweiten Stufe erhielt neben der ölsäurehaltigen organischen Phase vom Separator der ersten Stufe 7,0 kg der wie oben beschriebenen Pufferlösung, 2 kg Entleerungprodukt aus der zweiten Separatorstufe der Startphase und 5g frische Lipase des obigen Typs zur Nachschärfung. Ansonsten wurde wie in der Fettspaltung der ersten Stufe vorgegangen mit dem Ergebnis, daß auf der Seite der leichteren Phase des Separators der zweiten Stufe eine klare rohe Ölsäure-Phase mit einer Säurezahl von 184 ablief entsprechend einem Spaltgrad von 93% (berechnet als Quotient der gemessenen Säurezahl durch die sich für die vorliegende Mischung ergebende theoretische Säurezahl). Als schwere Phase wurde mit der Zentrifuge eine 12 Gew.%ige Glycerin-Wasser/Pufferlösung abgetrennt. Die so in der zweiten Stufe gewonnene Roh-Fettsäure wurde in einem als Zwischenbehälter dienenden 200 Liter-Faß gesammelt und einer Kurzwegdestillation auf einer Vakuum-Kurzwegdestille vom Typ KD 10 der Firma UIC, Alzenau-Hörstein, mit vorgeschalteter Entgasungsstufe zur Abtrennung etwaiger geringer Wassermengen unterzogen. Beispielsweise wurde dann bei einem Gesamtdruck von 0,014 mbar und 191 °C destilliert, wobei ca. 8 Gew.% der eingesetzten Fettsäure kontinuierlich als Rückstandsprodukt auf der Seite der hierbei nicht siedenden Bestandteile abliefen. Die anderen 92 Gew.% des Rohproduktes destillierten als Ölsäure mit einer Säurezahl von 199-200 und nur geringsten Mengen an Begleitstoffen in Form von Fettsäuren mit niedrigerem Dampfdruck. Dies entsprach im Rahmen der Meßgenauigkeit der theoretisch berechneten Säurezahl. (Der Wert war aufgrund des geringen Anteils an leichter flüchtigen kürzeren Fettsäuren etwas höher als die theoretische Säurezahl).

Das Rückstandsprodukt wurde einer Entwachsung unterzogen, wobei die enthaltenen höheren Fettsäuren ( C22 und größere Kettenlängen ), Wachse und andere höher siedenden Begleitstoffe des Öls als Feststoffe aus kristallisierten und mittels Filtration abgetrennt wurden. Das filtrierte Rückstandsprodukt wurde mit einer in FIG.1 angedeuteten Pumpe in den Spaltprozeß vor die erste Stufe zurückgeleitet.

Auf gleiche Weise wurden etliche weitere Beispiel gefahren, u.a. mit höheren Enzymmengen und entsprechend kürzeren Spaltzeiten, mit Rindertalg sowie mit Crambeöl. Bei dem Beispiel mit Crambeöl, das 60% Eurucasäure enthielt, wurde erfolgreich eine Mischung des obigen unspezifischen Enzyms OF 360 und des 1,3-spezifischen Enzym Novozym 388 verwendet, das speziell spezifisch die an der 1,3 Stellung des Triglyceridgerüsts sitzenden Fettsäuren abspaltet, in diesem Fall die Erucasäure.

## Patentansprüche

1. Verfahren zur enzymatischen Spaltung von Ölen und/oder Fetten bei gleichzeitiger enzymatischer Bildung von Fettsäureestern unter Einsatz von als Biokatalysatoren wirkenden Lipasen und von Alkoholen, insbesondere n- und Iso-Alkoholen, in welchem:

man auf ein Gemisch aus Triglyceriden, Wasser und einem öl- bzw. fettlöslichen Alkohol als Biokatalysatoren für eine Spaltung des Öls bzw. Fetts und eine Bildung von Fettsäureestern Lipasen einwirken läßt, das hierbei gebildete Reaktionsgemisch zur Trennung in eine wässrige, glycerinhaltige Phase und eine organische Phase, die die gebildeten Fettsäureester enthält, in eine selbstaustragende Zentrifuge gegeben wird, die Zentrifuge so eingestellt wird, daß eine zwischen der ablaufenden wässrigen und ablaufenden organischen Phase auftretende, mit Lipase angereicherte Zwischenschicht sich in der Zentrifuge ansammelt, und die Zentrifuge zu vorgegebenen Zeitpunkten entleert wird und der dabei ausgetragene Trommelinhalt der Zentrifuge in den kombinierten Spalt- und Esterbildungsprozeß zurückgeführt wird bzw. für einen weiteren Spalt- und Esterbildungsprozeß bereitgestellt wird.

2. Verfahren nach Anspruch 1,
**dadurch gekennzeichnet,**
**daß** der Alkohol mit einem Überschuß von 2 bis 100 %, vorzugsweise 5 bis 20%, gegenüber der stöchiometrisch erforderlichen Menge für die Esterbildung zugesetzt wird.

3. Verfahren nach Anspruch 1 oder 2,
**dadurch gekennzeichnet,**
**daß** das Wasser zugesetzt wird mit mindestens 5 Gew.% bezogen auf die eingesetzte organische Phase aus Öl bzw. Fett und Alkohol.

4. Verfahren nach einem der vorhergehenden Ansprüche,
**dadurch gekennzeichnet,**
**daß** als der Alkohol solche Alkohole eingesetzt werden, die in der gebildeten organischen Phase gut löslich, hingegen in Wasser erheblich schlechter löslich sind, insbesondere mittel bis langkettige n- und Iso-Alkohole.

5. Verfahren nach einem der vorhergehenden Ansprüche,
**dadurch gekennzeichnet,**
**daß** die Fettspaltung/Esterbildung diskontinuierlich in Reaktoren durchgeführt wird, die im Schlaufenbetrieb mit durch Pumpen umgewälztem Reaktorinhalt betrieben werden, wobei mehrere Reaktoren parallel für eine einzige oder jede mehrerer Reaktionsstufen vorgesehen sind, einer der Reaktoren bei nicht aktivierter Umwälzschlaufe befüllt wird, währenddessen ein weiterer Reaktor mit aktiver Umwälzschlaufe im Spalt/Esterbildungsbetrieb gefahren wird und ein noch weiterer Reaktor ebenfalls bei nicht aktivierter Umwälzschlaufe über eine Zentrifuge entleert wird, die sich beim Spaltprozeß bildende wässrige, glycerinhaltige Phase von der organischen Phase, die Fettsäureester enthält, trennt, bevor die Fettsäureester von der organischen Phase getrennt werden.

6. Verfahren nach einem der vorhergehenden Ansprüche,
**dadurch gekennzeichnet,**
**daß** als Lipasen unspezifische Lipasen, spezifische Lipasen oder Mischungen von unspezifischen und spezifischen Lipasen eingesetzt werden.

7. Verfahren nach einem der vorhergehenden Ansprüche,
**dadurch gekennzeichnet,**
**daß** aus der organischen Phase freie Fettsäuren und Alkohol von den gebildeten Fettsäureestern destillativ abgetrennt werden und in den Spalt/Esterbildungsprozeß zurückgeführt werden.

8. Verfahren nach einem der vorhergehenden Ansprüche,
**dadurch gekennzeichnet,**
**daß** die aus der selbstaustragenden Zentrifuge abfließende organische Phase einer weiteren selbstaustragenden Zentrifuge, insbesondere einer Polierzentrifuge zugeführt wird, die ebenfalls intermittierend entleert wird, um die sich als Sediment an der Zentrifugenwand abscheidenden Lipaserestmengen für den Spaltprozeß wieder zu gewinnen.

9. Vorrichtung zur Durchführung des Verfahrens nach einem der vorhergehenden Ansprüche, aufweisend:

einen oder mehrere Spalt/Esterbildungsreaktoren,
eine oder mehrere selbstaustragende Zentrifugen, in denen sich die zwischen der abfließenden wässrigen und abfließenden organischen Phase auftretende, mit Lipase angereicherte Zwischenschicht ansammelt, und die zu vorgegebenen Zeitpunkten entleert werden,
eine Rückführungseinrichtung zur Rückführung des intermittierend ausgetragenen Trommelinhalts der Zentri-

fuge in den kombinierten Spalt- und Esterbildungsprozeß, und eine
Einrichtung zur Trennung von Alkohol, freien Fettsäuren und gebildeten Fettsäureestern aus der von der Zentrifuge gelieferten organischen Phase.

**10.** Vorrichtung nach Anspruch 9,
**dadurch gekennzeichnet,**
**daß** die Einrichtung zur Trennung eine Destillationseinrichtung, insbesondere eine Kurzwegdestille oder ein Fallfilmverdampfer, ist.

**11.** Verfahren zur enzymatischen Spaltung von Ölen und/oder Fetten unter Einsatz von als Biokatalysatoren wirkenden Lipasen zur Gewinnung von Fettsäuren und Glycerin, in welchem:

man auf ein Gemisch aus einem Öl bzw. Fett und Wasser als Biokatalysatoren für eine Spaltung des Öls bzw. Fetts Lipasen einwirken läßt,
das hierbei gebildete Reaktionsgemisch zur Trennung in eine wässrige, glycerinhaltige Phase und eine organische Phase, die durch die vorausgehende Spaltung abgespaltene freie Fettsäuren enthält, in eine selbstaustragende Zentrifuge gegeben wird,
die Zentrifuge so eingestellt wird, daß eine zwischen der ablaufenden wässrigen und ablaufenden organischen Phase auftretende, mit Lipase angereicherte Zwischenschicht sich in der Zentrifuge ansammelt, und
die Zentrifuge zu vorgegebenen Zeitpunkten entleert wird und der dabei ausgetragene Trommelinhalt der Zentrifuge in den Spaltprozeß zurückgeführt wird bzw. für einen weiteren Spaltprozeß bereitgestellt wird.

**12.** Verfahren nach Anspruch 11,
**dadurch gekennzeichnet,**
**daß** die die Fettspaltung diskontinuierlich in Reaktoren durchgeführt wird, die im Schlaufenbetrieb mit durch Pumpen umgewälztem Reaktorinhalt betrieben werden, wobei mehrere Reaktoren parallel für eine einzige oder jede mehrerer Reaktionsstufen vorgesehen sind, einer der Reaktoren bei nicht aktivierter Umwälzschlaufe befüllt wird, währenddessen ein weiterer Reaktor mit aktiver Umwälzschlaufe im Spaltbetrieb gefahren wird und ein noch weiterer Reaktor ebenfalls bei nicht aktivierter Umwälzschlaufe über eine Zentrifuge entleert wird, die sich beim Spaltprozeß bildende wässrige, glycerinhaltige Phase von der organischen Phase, die freie Fettsäuren enthält, trennt, bevor die Fettsäuren von der organischen Phase getrennt werden.

**13.** Verfahren nach einem der Ansprüche 11 und 12,
**dadurch gekennzeichnet,**
**daß** aus der organischen Phase freie Fettsäuren und Alkohol von den gebildeten Fettsäureestern destillativ abgetrennt werden und in den Spalt/Esterbildungsprozeß zurückgeführt werden.

**14.** Verfahren nach Anspruch 13,
**dadurch gekennzeichnet,**
**daß** die Trennung in einer Destillationseinrichtung, insbesondere einer Kurzwegdestille oder einem Fallfilmverdampfer, erfolgt.

**Claims**

**1.** A method for enzymatically splitting oils and/or fats under simultaneous enzymatic formation of fatty acid esters using lipases acting as biocatalysts and alcohols, in particular n- and iso-alcohols, wherein:

lipases are caused to act on a mixture of triglycerides, water and an oil- or fat-soluble alcohol as biocatalysts for splitting the oil or fat and for forming fatty acid esters,
the reaction mix formed thereby is, for separation into an aqueous, glycerine-containing phase and an organic phase containing the fatty acid esters formed, transferred into a self-discharging centrifuge,
the centrifuge is set up in such a way that a lipase-enriched intermediate layer present between the discharging aqueous and the discharging organic phases collects in the centrifuge, and
the centrifuge is emptied at specified times and the drum content of the centrifuge which is discharged thereby is recycled back into the combined splitting and ester formation process or is made available for a further splitting and ester formation process.

**2.** The method as claimed in claim 1, **characterised in that** the alcohol is added at an excess of 2 to 100 %, preferably of 5 to 20 %, in relation to the amount stoichiometrically required for the ester formation.

**3.** The method as claimed in claim 1 or 2, **characterised in that** the water added is at least 5 % by weight in relation to the used organic phase of oil or fat and alcohol.

**4.** The method as claimed in any one of the preceding claims, **characterised in that** as the alcohol such alcohols are used which are highly soluble in the organic phase formed, but are considerably less soluble in water, in particular medium- and long-chained n- and iso-alcohols.

**5.** The method as claimed in any one of the preceding claims, **characterised in that** the fat splitting / ester formation is carried out discontinuously in reactors operated in a loop mode having a reactor content which is circulated by pumps, with several reactors being provided in parallel for one single or each of several reactor stage(s), with one of the reactors being filled during a non-activated circulation loop, whereas a further reactor with an active circulation loop is run in a splitting/ester formation mode of operation and a still further reactor is also emptied in a non-activated circulation loop via a centrifuge which separates any aqueous, glycerine-containing phase forming during the splitting process from the organic phase containing fatty acid ester, before the fatty acid esters are separated from the organic phase.

**6.** The method as claimed in any one of the preceding claims, **characterised in that** as lipases, unspecific lipases, specific lipases or mixtures of unspecific and specific lipases are used.

**7.** The method as claimed in any one of the preceding claims, **characterised in that** free fatty acids and alcohols from the fatty acid esters formed are separated from the organic phase by distillation and are recycled back into the splitting/ester formation process.

**8.** The method as claimed in any one of the preceding claims, **characterised in that** the organic phase discharging from the self-discharging centrifuge is fed towards a further self-discharging centrifuge, in particular a polishing centrifuge which is also emptied intermittently, in order to recover the residue lipase amounts separating on the wall of the centrifuge for the splitting process.

**9.** A. device for carrying out the method as claimed in any one of the preceding claims, comprising:

one or several splitting/ester formation reactors,
one or several self-discharging centrifuges in which the lipase-enriched intermediate layer present between the discharging aqueous and the discharging organic phases collects and which are emptied at specified times,
a recycling device for recycling the intermittently discharged drum content of the centrifuge back into the combined splitting and ester formation process, and
a device for separating alcohols, free fatty acids and fatty acid esters formed from the organic phase supplied from the centrifuge.

**10.** The device as claimed in claim 9, **characterised in that** the device for separating is a distillation device, in particular a short-path distiller or a falling film evaporator.

**11.** A method for enzymatically splitting oils and/or fats using lipases acting as biocatalysts for obtaining fatty acids and glycerine, wherein:

lipases are caused to act on a mixture of an oil or a fat and water as biocatalysts for the splitting of the oil or fat, the reaction mix formed thereby is, for the separation into an aqueous, glycerine-containing phase and an organic phase containing the free fatty acids split off in the preceding splitting process, transferred to a shelf-discharging centrifuge,
the centrifuge is set up in such a way that a lipase-enriched intermediate layer present between the discharging aqueous and the discharging organic phases collects in the centrifuge, and
the centrifuge is emptied at specified times and the drum content of the centrifuge which is discharged thereby is recycled back into the splitting process or is made available for a further splitting process.

**12.** The method as claimed in claim 11, **characterised in that** the fat splitting is carried out discontinuously in reactors operated in a loop mode having a reactor content which is circulated by pumps, with several reactors being provided in parallel for one single or each of several reactor stage(s), with one of the reactors bering filled during a non-activated circulation loop, whereas a further reactor with an active circulation loop being run in a splitting mode of operation and a still further reactor is also emptied in a non-activated circulation loop via a centrifuge which separates any aqueous, glycerine-containing phase forming during the splitting process from the organic phase containing fatty acids, before the fatty acids are separated from the organic phase.

13. The method as claimed in any one of claims 11 and 12, **characterised in that** from the organic phase, free fatty acids and alcohol are separated from the fatty acid esters formed by distillation and are recycled back into the splitting/ester formation process.

14. The method as claimed in claim 13, **characterised in that** the separation is carried out in the distillation device, in particular a short-path distiller or a falling film evaporator.

**Revendications**

1. Procédé de clivage enzymatique d'huiles et/ou graisses avec formation enzymatique simultanée d'esters d'acide gras par mise en oeuvre de lipases agissant comme biocatalyseurs, et d'alcools, en particulier des n- et iso-alcools, dans lequel :

on fait agir sur un mélange de triglycérides, d'eau et d'un alcool soluble dans les huiles et respectivement, les graisses, des lipases comme biocatalyseurs pour le clivage des huiles et respectivement graisses, et la formation d'esters d'acide gras,
le mélange réactionnel ainsi formé est disposé dans une centrifugeuse à décharge automatique pour la séparation en une phase aqueuse, contenant la glycérine et une phase organique, qui contient les esters d'acide gras formés,
la centrifugeuse est réglée de sorte qu'une couche intermédiaire enrichie en lipase, se trouvant entre la phase aqueuse s'écoulant et la phase organique s'écoulant, se rassemble dans la centrifugation, et
la centrifugeuse est vidée en des moments prédéterminés, et le contenu du tambour alors extrait de la centrifugeuse est ramené dans le processus combiné de clivage et de formation d'ester et respectivement, préparé pour un autre processus de clivage et de formation d'ester.

2. Procédé selon la revendication 1,
**caractérisé en ce**
**que** l'alcool est ajouté en un excès allant de 2 à 100%, de préférence de 5 à 20%, par rapport à la quantité stoechiométriquement nécessaire pour la formation de l'ester.

3. Procédé selon la revendication 1 ou 2,
**caractérisé en ce**
**que** l'eau est ajoutée à au moins 5% en poids par rapport à la phase organique mise en oeuvre, constituée de l'huile et respectivement, graisse et de l'alcool.

4. Procédé selon l'une des revendications précédentes,
**caractérisé en ce**
**que** l'on met en oeuvre comme alcool, des alcools qui sont bien solubles dans la phase organique formée, qui sont par contre, bien moins soluble dans l'eau, en particulier des n- et iso-alcools à chaîne moyenne à longue.

5. Procédé selon l'une des revendications précédentes,
**caractérisé en ce**
**que** le clivage de graisse/formation d'ester est réalisé de manière discontinue dans des réacteurs, qui sont actionnés en boucle avec le contenu du réacteur circulant par des pompes, où plusieurs réacteurs sont prévus en parallèle pour une seule ou chacune de plusieurs étapes de réaction, un des réacteurs est rempli lors d'une boucle de circulation non activée, pendant ce temps, un autre réacteur procède à une boucle de circulation active en mode clivage/formation d'ester et un autre réacteur encore est vidé par une centrifugeuse également lors d'une boucle de circulation non activée, la centrifugeuse séparant la phase aqueuse, contenant la glycérine, de la phase organique, qui contient les esters d'acide gras, avant de séparer les esters d'acide gras de la phase organique.

6. Procédé selon l'une des revendications précédentes,

**caractérisé en ce**
**que** l'on met en oeuvre comme lipases, des lipases non spécifiques, des lipases spécifiques ou des mélanges de lipases non spécifiques et spécifiques.

**7.** Procédé selon l'une des revendications précédentes,
**caractérisé en ce**
**que** dans la phase organique, les acides gras et alcools libres sont séparés des esters d'acide gras formés par distillation et ramenés dans le processus de clivage/formation d'ester.

**8.** Procédé selon l'une des revendications précédentes,
**caractérisé en ce**
**que** la phase organique s'écoulant de la centrifugeuse à décharge automatique, est alimentée dans une autre centrifugeuse à décharge automatique, en particulier une centrifugeuse de finition, qui est également vidée de manière intermittente, pour récupérer les quantités résiduelles de lipases se déposant comme sédiment sur la paroi de la centrifugeuse, pour le processus de clivage.

**9.** Dispositif pour réaliser le procédé selon l'une des revendications précédentes, présentant :

   un ou plusieurs réacteurs de clivage/formation d'ester,
   une ou plusieurs centrifugeuses à décharge automatique, dans lesquelles la couche intermédiaire enrichie en lipase, se présentant entre la phase aqueuse et la phase organique se rassemble et qui sont vidées en des moments prédéterminés,
   un dispositif de recyclage pour le recyclage du contenu du tambour de la centrifugeuse, extrait de manière intermittente, dans le processus combiné de clivage et de formation d'ester, et
   un dispositif pour la séparation de l'alcool, des acides gras libres et des esters d'acide gras formés, dans la phase organique provenant de la centrifugeuse.

**10.** Dispositif selon la revendication 9,
**caractérisé en ce**
**que** le dispositif pour la séparation est un dispositif de distillation, en particulier une distillation flash ou un évaporateur à couche mince.

**11.** Procédé de clivage enzymatique d'huiles et/ou graisses avec mise en oeuvre de lipases agissant comme biocatalyseurs, pour l'obtention d'acides gras et de glycérine, dans lequel :

   on fait agir sur un mélange d'une huile et respectivement, graisse et d'eau, des lipases comme biocatalyseurs pour le clivage des huiles et respectivement graisses,
   le mélange réactionnel ainsi formé est disposé dans une centrifugeuse à décharge automatique pour la séparation en une phase aqueuse, contenant la glycérine et une phase organique, qui contient les acides gras libres obtenus du clivage précédent,
   la centrifugeuse est réglée de sorte qu'une couche intermédiaire enrichie en lipase, se trouvant entre la phase aqueuse s'écoulant et la phase organique s'écoulant, se rassemble dans la centrifugation, et
   la centrifugeuse est vidée en des moments prédéterminés, et le contenu du tambour alors extrait de la centrifugeuse est ramené dans le processus de clivage et respectivement, préparé pour un autre processus de clivage.

**12.** Procédé selon la revendication 11,
**caractérisé en ce**
**que** le clivage de graisse est réalisé de manière discontinue dans des réacteurs, qui sont actionnés en boule avec le contenu du réacteur circulant par des pompes, où plusieurs réacteurs sont prévus en parallèle pour une seule ou chacune de plusieurs étapes de réfaction, un des réacteurs est rempli lors d'une boucle de circulation non activée, pendant ce temps, un autre réacteur procède à une boucle de circulation active en mode clivage est un autre réacteur encore est vidé par une centrifugeuse également lors d'une boucle de circulation non activée, la centrifugeuse séparant la phase aqueuse, contenant la glycérine, se formant lors du processus de clivage, de la phase organique, qui contient les acides gras libres, avant de séparer les acides gras de la phase organique.

**13.** Procédé selon l'une des revendications 11 et 12,
**caractérisé en ce**
**que** dans la phase organique, les acides gras libres et l'alcool sont séparés par distillation des esters d'acide gras formés et ramenés dans le processus de clivage/formation d''ester.

14. Procédé selon la revendication 13,
   **caractérisé en ce**
   **que** la séparation est réalisée dans un dispositif de distillation, en particulier une distillation flash ou un évaporateur à couche mince.

Fig. 1 a

Fig. 1 b

FIG. 2

Fettspaltung
(enzymatisch)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0195311 A

**In der Beschreibung aufgeführte Nicht-Patentliteratur**

- **M. Bühler ; Chr. Wandrey.** Continuous Use of Lipases in Fat Hydrolysis. *Fat Science Technology 89,* vol. 87, 598-605
- **M. Bühler ; Chr. Wandrey.** Enzymatische Fettspaltung. *Fett Wissenschaft Technologie 89,* 1987, 156-164
- **M. Bühler ; Chr. Wandrey.** Oleochemicals by Biochemical Reactions ?. *Fat Science Technology 94,* 1992, 82-94